(19) Europäisches Patentamt
European Patent Office
Office européen des brevets

(11) **EP 2 379 222 B1**

(12) **EUROPÄISCHE PATENTSCHRIFT**

(45) Veröffentlichungstag und Bekanntmachung des Hinweises auf die Patenterteilung:
**05.09.2018 Patentblatt 2018/36**

(51) Int Cl.:
**B01J 27/198** (2006.01)     **B01J 35/02** (2006.01)
**C07C 51/215** (2006.01)     **B01J 35/10** (2006.01)

(21) Anmeldenummer: **09795778.1**

(22) Anmeldetag: **21.12.2009**

(86) Internationale Anmeldenummer:
**PCT/EP2009/067661**

(87) Internationale Veröffentlichungsnummer:
**WO 2010/072723 (01.07.2010 Gazette 2010/26)**

(54) **KATALYSATOR UND VERFAHREN ZUR HERSTELLUNG VON MALEINSÄUREANHYDRID**

CATALYST AND METHOD FOR PRODUCING MALEIC ANHYDRIDE

CATALYSEUR ET PROCÉDÉ DE PRODUCTION D'ANHYDRIDE MALÉIQUE

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **22.12.2008 EP 08172635**

(43) Veröffentlichungstag der Anmeldung:
**26.10.2011 Patentblatt 2011/43**

(73) Patentinhaber: **BASF SE**
**67056 Ludwigshafen am Rhein (DE)**

(72) Erfinder:
• **DOBNER, Cornelia, Katharina**
**67071 Ludwigshafen (DE)**
• **ALTWASSER, Stefan**
**67157 Wachenheim (DE)**
• **WILMER, Hagen**
**67071 Ludwigshafen (DE)**
• **ROSOWSKI, Frank**
**68239 Mannheim (DE)**

(56) Entgegenhaltungen:
**EP-A- 1 120 390     EP-A- 1 386 664
WO-A-02/34387     WO-A-2007/051602
JP-A- 6 170 239**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die vorliegende Erfindung betrifft einen Katalysator zur Herstellung von Maleinsäureanhydrid (MSA) durch Gasphasenpartialoxidation. Ferner betrifft die Erfindung ein Verfahren zur Herstellung von Maleinsäureanhydrid unter Verwendung des erfindungsgemäßen Katalysators.

Zur heterogen katalysierten Gasphasenoxidation leitet man im Allgemeinen ein Gemisch aus einem sauerstoffhaltigen Gas, beispielsweise Luft, und dem zu oxidierenden Kohlenwasserstoff durch eine Vielzahl in einem Reaktor angeordneter Rohre, in denen sich eine Schüttung von Katalysatorformkörpem befindet.

Heterogene Katalysatoren auf der Basis von Vanadylpyrophosphat $(VO)_2P_2O_7$ (so genannte VPO-Katalysatoren) werden bei der industriellen Oxidation von Kohlenwasserstoffen, wie z. B. n-Butan, zu Maleinsäureanhydrid eingesetzt. Die Geschwindigkeit dieser heterogen katalysierten Oxidation in der Gasphase ist durch so genannte innere Transporteinflüsse limitiert. Hierbei begrenzt nicht die Diffusion der Reaktanden an die äußere Oberfläche des Katalysators die Umsetzungsgeschwindigkeit, sondern die Diffusionsgeschwindigkeit im Porengefüge des Katalysators stellt den limitierenden Schritt dar.

Die Stofftransportlimitierung kann durch Erhöhung der Porosität des Katalysators verringert werden. Hierzu werden der katalytisch aktiven Masse Porenbildner zugesetzt, d. h. organische Verbindungen, die beim späteren Erhitzen verbrennen. Dieser Optimierung sind jedoch Grenzen gesetzt, weil übermäßig poröse Katalysatorformkörper keine ausreichende mechanische Stabilität mehr aufweisen.

Eine weitere Möglichkeit zur Verbesserung dieser Katalysatoren bietet die Optimierung der Formkörpergeometrie. Diese bestimmt sowohl die äußere Oberfläche des Katalysators als auch den Widerstand, den die Katalysatorteilchen dem durchströmenden Gas entgegensetzen und somit das Druckgefälle, das zwischen dem Reaktoreingang und dem Reaktorausgang überwunden werden muss. Durch Verwendung kleinerer Katalysatorteilchen vergrößert sich zwar die äußere Oberfläche und damit die Aktivität des Katalysators, gleichzeitig steigt aber der Druckverlust stark an. Der Variation der Formkörpergeometrie sind außerdem insofern Grenzen gesetzt, dass der erhaltene Katalysator eine ausreichende mechanische Stabilität aufweisen und z. B. beim Einfüllen in die Reaktionsrohre kein übermäßiger Bruch auftreten soll. Geometrien mit weit hervorstehenden Teilen, dünnen Stegen und dergleichen weisen im Allgemeinen eine unzureichende mechanische Stabilität auf.

Es wird also eine Katalysatorgeometrie angestrebt, die eine große äußere Oberfläche, einen geringeren Druckverlust und ausreichende mechanische Stabilität aufweist.

Aus der US 4,283,307 geht ein Oxidationskatalysator für die Partialoxidation von n-Butan zu MSA in Form einer Tablette mit Mittelloch hervor.

Die US 5,168,090 beschreibt Katalysatorformkörper für die Herstellung von MSA, deren äußere Oberfläche wenigstens einen Hohlraum aufweist und deren geometrisches Volumen 30 bis 67 % des Volumens der hohlraumfreien geometrischen Form entspricht und die ein Verhältnis der äußeren geometrischen Oberfläche zum geometrischen Volumen von wenigstens 20 cm$^{-1}$ aufweisen. Speziell veranschaulicht die US 5,168,090 Zylinder mit 3 äquidistanten Nuten in der äußeren Oberfläche, die parallel zur Zylinderachse verlaufen.

Die WO 01/68245 offenbart einen Katalysator für die Herstellung von Maleinsäureanhydrid durch heterogenkatalytische Gasphasenoxidation, der eine im wesentlichen hohlzylinderförmige Struktur aufweist, wobei die hohlzylinderförmige Struktur ein bestimmtes Verhältnis der Höhe zum Durchmesser der hindurchgehenden Öffnung und ein bestimmtes Verhältnis der geometrischen Oberfläche zum geometrischen Volumen aufweist.

Die WO 03/078057 beschreibt einen Katalysator für die Herstellung von Maleinsäureanhydrid, der eine katalytisch aktive Masse enthaltend Vanadium, Phosphor und Sauerstoff umfasst und eine im Wesentlichen hohlzylinderförmige Struktur aufweist, dessen geometrische Dichte $d_p$ bestimmten Bedingungen genügt.

Die WO 2007/051 602 beschreibt Katalysatorformkörper zur Herstellung von Maleinsäureanhydrid, wobei der den Katalysatorformkörper umhüllende geometrische Grundkörper ein Prisma ist und der Katalysatorformkörper mit drei durchgehenden Öffnungen versehen ist. Der Katalysatorformkörper soll einen dreieckigen Querschnitt mit abgerundeten Scheiteln aufweisen.

Die EP-A 1 120 390 beschreibt ein Verfahren zur Herstellung von 1,2-Dichlorethan durch Oxychlorierung von Ethen an einer Schüttung aus Katalysatorpartikeln. Die Katalysatorpartikel können als Zylinder mit axialen Bohrungen vorliegen.

Die JP 06-170239 offenbart Katalysatorformkörper für die Synthese ungsättigter Aldehyde und Carbonsäuren mit einem zylindrischen Körper und wenigstens zwei Bohrungen.

Der vorliegenden Erfindung liegt die Aufgabe zugrunde, einen Katalysatorformkörper für die Herstellung von MSA aufzuzeigen, der eine große äußere Oberfläche, einen geringeren Druckverlust und ausreichende mechanische Stabilität aufweist. Insbesondere liegt der Erfindung die Aufgabe zugrunde, einen Katalysatorformkörper aufzuzeigen, der eine hohe Ausbeute bei der Gasphasenpartialoxidation und hohe Selektivität bezüglich MSA bei geringem Druckverlust ermöglicht.

Erfindungsgemäß wird die Aufgabe gelöst durch einen Katalysatorformkörper zur Herstellung von Maleinsäureanhydrid durch Gasphasenoxidation eines Kohlenwasserstoffs mit mindestens vier Kohlenstoffatomen mit einer katalytisch aktiven Masse, die Vanadium, Phosphor und Sauerstoff enthält, wobei der Katalysatorformkörper einen im wesentlichen zylindrischen Körper mit einer Längsachse aufweist, wobei der zylindrische Körper vier zur Zylinderachse des Körpers im wesentlichen parallele, durchgehende Innenbohrungen aufweist, die Summe der Mantelflächen der Innenbohrungen gleich oder größer als die äußere Mantelfläche des zylindrischen Körpers ist und sowohl der kleinste Abstand der Innenbohrungen untereinander als auch der kleinste Abstand der Innenbohrungen zur äußeren Mantelfläche des Körpers jeweils wenigstens 6% des Durchmessers des zylindrischen Körpers beträgt. Der Katalysatorformkörper weist einen wesentlichen zylindrischen Körper, d. h. einen Körper mit kreisförmigen Querschnitt und planparallelen Deckelflächen, auf. Der Begriff "im Wesentlichen" weißt darauf hin, dass Abweichungen von der Idealgeometrie, wie beispielsweise leichte Deformationen der kreisförmigen Struktur, nicht planparallel ausgerichtete Deckelflächen, abgeplatzte Ecken und Kanten, Oberflächenrauhigkeit oder Einkerbungen in der Mantelfläche, den Deckelflächen oder der Innenfläche der hindurchgehenden Bohrungen beim erfindungsgemäßen Katalysatorformkörper mit umfasst sind. Erfindungsgemäß weist der zylindrische Körper vier Innenbohrungen auf.

Die Innenbohrungen weisen vorzugsweise einen runden oder ovalen Querschnitt auf, insbesondere einen runden Querschnitt. Im Allgemeinen weisen alle Innenbohrungen den gleichen Querschnitt auf.

In bevorzugten Ausführungsformen liegen die Zentralachsen der Innenbohrungen äquidistant auf einem Zylindermantel, der konzentrisch zum Mantel des zylindrischen Körpers ist. Vorzugsweise beträgt das Verhältnis des Durchmessers $d_2$ einer Innenbohrung zum äußeren Durchmesser $d_1$ des zylindrischen Körpers 0,2 bis 0,35. Vorzugsweise beträgt das Verhältnis des Durchmessers $d_3$ des Zylindermantels, auf dem die Zentralachsen der Innenbohrungen liegen, zum äußeren Durchmesser $d_1$ des zylindrischen Körpers 0,53 bis 0,60.

Durch den Einsatz von zwei oder mehr Innenbohrungen kann gegenüber einem einfachen Hohlzylinder bei vergleichbarem Hohlraumanteil die Fläche, die durch die Mantelflächen der Innenbohrungen gebildet wird, stark vergrößert werden.

Erfindungsgemäß ist die Summe der Mantelflächen der Innenbohrungen gleich oder größer als die äußere Mantelfläche des zylindrischen Körpers.

Für den Erhalt einer ausreichenden mechanischen Stabilität beträgt sowohl der kleinste Abstand der Innenbohrungen untereinander als auch der kleinste Abstand der Innenbohrungen zur äußeren Mantelfläche des Körpers jeweils wenigstens 6, insbesondere wenigstens 7 % des Durchmessers $d_1$ des zylindrischen Körpers. Das Verhältnis der Höhe h des zylindrischen Körpers zum Durchmesser $d_2$ der Innenbohrungen beträgt vorzugsweise höchstens 3,5 insbesondere 2,2 bis 3,4. bitte prüfen, sollte bei 3,5 liegen.

Das Verhältnis der geometrischen Oberfläche $A_{geo}$ zum geometrischen Volumen $V_{geo}$ beträgt vorzugsweise mindestens 2,0 mm$^{-1}$, z. B. 2,05 bis 2,6 mm$^{-1}$ vorzugsweise 2,1 bis 2,4 mm$^{-1}$. Die geometrische Oberfläche $A_{geo}$ und das geometrische Volumen $V_{geo}$ ergibt sich aus den äußeren, makroskopischen Abmessungen des Katalysatorformkörpers unter Berücksichtigung des äußeren Durchmessers $d_1$, der Höhe h und des Durchmessers $d_2$ und Anzahl der Innenbohrungen.

Vorzugsweise beträgt das Verhältnis des geometrischen Volumens $V_{geo}$ des zylindrischen Körpers zum theoretischen Volumen $V_{overall}$ eines entsprechenden Vollzylinders mit gleicher Höhe h und gleichem äußeren Durchmesser $d_1$ höchstens 0,85, insbesondere 0,55 bis 0,72.

Im Allgemeinen beträgt der äußere Durchmesser $d_1$ des zylindrischen Körpers 3 bis 10 mm, bevorzugt 4 bis 8 mm, insbesondere 5 bis 7 mm, die Höhe h des zylindrischen Körpers 1 bis 10 mm, bevorzugt 2 bis 6 mm, insbesondere 3 bis 5 mm, und der Durchmesser jeder Innenbohrung $d_2$ 0,5 bis 4 mm, bevorzugt 1 bis 3 mm.

Der Katalysatorformkörper ist vorzugsweise porös und weist insbesondere ein spezifisches Porenvolumen von mindestens 0,30 ml/g, besonders bevorzugt mindestens 0,35 ml/g, z. B. von 0,38 bis 0,50 ml/g auf. Das spezifische Porenvolumen PV ist das durch Quecksilberporosimetrie gemäß DIN Nr. 66133 bestimmte (integrale) spezifische Porenvolumen.

Bei dem erfindungsgemäßen Katalysatorformkörper kann es sich um einen Vollkatalysator oder einen Mischkatalysator handeln. Unter einem Vollkatalysator wird ein Formkörper verstanden, der im Wesentlichen vollständig aus der katalytisch aktiven Masse besteht. Unter einem Mischkatalysator wird ein Formkörper verstanden, der die katalytisch aktive Masse in verdünnter Form im Gemisch mit einem Trägermaterial enthält. Als geeignete Trägermaterialien für die Mischkatalysatoren seien beispielsweise Aluminiumoxid, Siliziumdioxid, Alumosilikate, Zirkondioxid, Titandioxid oder Gemische davon genannt.

Zur Herstellung von Voll- oder Mischkatalysatoren wird eine katalytisch aktive Masse bzw. eine Vorläufermasse, die durch Calcinieren in eine katalytisch aktive Form überführt werden kann, gegebenenfalls im Gemisch mit einem pulverförmigen inerten Träger zu einem erfindungsgemäßen Katalysatorformkörper geformt.

Der zu formenden Masse können Porenbildner zugesetzt werden. Porenbildner sind Stoffe, welche zur gezielten Herstellung poröser Formkörper eingesetzt werden. In der Regel handelt es sich um Kohlenstoff, Wasserstoff, Sauerstoff und/oder Stickstoff enthaltende Verbindungen, welche vor der Formgebung des Katalysator zugesetzt werden und bei der anschließenden Aktivierung des Katalysators unter Sublimation, Zersetzung und/oder Verdampfung zum überwiegenden Teil wieder entfernt werden. Der fertige Katalysator kann Rückstände oder Zersetzungsprodukte des Porenbildners enthalten. Geeignete Porenbildner sind z. B. Fettsäuren, wie Palmitinsäure oder Stearinsäure, Dicarbonsäuren, wie Oxalsäure oder Malonsäure, Cyclodextrine oder Polyethylenglykole. Die Verwendung von Malonsäure ist bevorzugt. Falls verwendet, wird der Porenbildner vorzugsweise in einer Menge von 16 bis 40 Gew.-Teilen, insbesondere 20 bis 25 Gew.-Teilen, bezogen auf 100 Gew.-Teile der katalytisch aktiven Masse eingesetzt.

Die erfindungsgemäßen Katalysatorformkörper besitzen vorteilhaft eine BET-Oberfläche von mehr als 15 $m^2$/g, bevorzugt von mehr als 15 bis 50 $m^2$/g und insbesondere von mehr als 15 bis 40 $m^2$/g. Sie weisen vorteilhaft ein Porenvolumen von mehr als 0,1 ml/g auf, bevorzugt von 0,15 bis 0,6 ml/g und insbesondere von 0,3 bis 0,5 ml/g.

Die Formgebung erfolgt vorzugsweise durch Tablettierung. Die Tablettierung ist ein Verfahren der Pressagglomeration. Dabei wird ein pulverförmiges oder vorab agglomeriertes Schüttgut in ein Presswerkzeug mit einer so genannten Matrize zwischen zwei Stempeln eingefüllt und durch einachsige Kompression verdichtet und zu einem festen Komprimat geformt. Dieser Vorgang gliedert sich in vier Abschnitte: Dosierung, Verdichtung (elastische Verformung), plastische Verformung und Ausstoßen. Die Tablettierung erfolgt z. B. auf so genannten Rundläuferpressen oder Exzenterpressen.

Zur Ausbildung der Innenbohrungen weist der Oberstempel und/oder Unterstempel herausragende Dorne auf. Es ist auch möglich, die Pressstempel mit mehreren beweglichen Dornen auszustatten, so dass ein Stempel beispielsweise aus fünf Teilstempeln ("Ringstempel" mit vier "Löchern" und vier Dornen) aufgebaut sein kann.

Die Presskraft bei der Tablettierung bewirkt die Verdichtung des Schüttguts. In der Praxis hat es sich bewährt, die sogenannte Seitendruckfestigkeit der Formlinge durch Wahl der entsprechenden Presskraft gezielt einzustellen und stichprobenartig zu überprüfen. Unter der Seitendruckfestigkeit ist jene Kraft zu verstehen, bei der der Katalysatorformkörper, welcher sich zwischen zwei planparallelen Platten befindet, bricht, wobei die beiden planparallelen Deckelflächen des zylindrischen Körpers hierbei im rechten Winkel zu den planparallelen Platten stehen.

Bei der Tablettierung können Tablettierhilfsmittel, wie Graphit oder Magnesiumstearat mitverwendet werden. Die Verwendung eines Graphits mit einer spezifische Oberfläche von 0,5 bis 5 $m^2$/g und eines Partikeldurchmessers $d_{50}$ von 40 bis 200 $\mu$m, wie in der WO 2008/087116 beschrieben, ist bevorzugt.

Als Alternative zur Tablettierung sei beispielsweise die Extrusion genannt. Bei dieser Variante teigt man beispielsweise das Schüttgut an, um eine extrusionsfähige Masse zu erhalten. Diese kann dann zu einem Strang mit mehreren Kanälen im Inneren extrudiert und der Strang in zylindrische Stücke geschnitten werden.

Die katalytisch aktive Masse enthält Vanadium, Phosphor und Sauerstoff. Das Phosphor/Vanadium-Atomverhältnis beträgt im Allgemeinen 0,9 bis 1 ,5, bevorzugt 0,9 bis 1,2, insbesondere 1,0 bis 1,1. Die mittlere Oxidationsstufe des Vanadiums beträgt vorzugsweise +3,9 bis +4,4 und bevorzugt 4,0 bis 4,3. Geeignete Aktivmassen sind beispielsweise in den Patentschriften US 5,275,996, US 5,641,722, US 5,137,860, US 5,095,125 oder US 4,933,312 beschrieben.

Die erfindungsgemäßen Katalysatoren können des Weiteren sogenannte Promotoren enthalten. Als geeignete Promotoren sind die Elemente der 1. bis 15. Gruppe des Periodensystems sowie deren Verbindungen genannt. Geeignete Promotoren sind beispielsweise in den Offenlegungsschriften WO 97/12674 und WO 95/26817 sowie in den Patenten US 5,137,860, US 5,296,436, US 5,158,923 und US 4,795,818 beschrieben. Bevorzugt werden als Promotoren Verbindungen der Elemente Kobalt, Molybdän, Eisen, Zink, Hafnium, Zirkon, Lithium, Titan, Chrom, Mangan, Nickel, Kupfer, Bor, Silicium,. Antimon, Zinn, Niob und Wismut, besonders bevorzugt Molybdän, Eisen, Zink, Antimon, Wismut, Lithium. Die promotierten erfindungsgemäßen Katalysatoren können einen oder mehrere Promotoren enthalten. Der Gehalt an Promotoren beträgt in Summe im fertigen Katalysator im Allgemeinen nicht mehr als etwa 5 Gew.-%, jeweils als Oxid gerechnet. Bevorzugt sind solche Massen, die keine Promotoren enthalten, und solche, die Eisen oder Molybdän als Promotor enthalten.

Die wesentlichen Schritte der bevorzugten Katalysatorherstellung unter Bildung eines sogenannten Precursorpulvers, Formgebung und anschließende Calcinierung sind im Folgenden erläutert.

(a) Umsetzung einer fünfwertigen Vanadiumverbindung mit einem organischen, reduzierenden Lösungsmittel in Gegenwart einer Phosphorverbindung unter Erwärmen. Gegebenenfalls kann diese Stufe in Gegenwart eines dispergierten, pulverförmigen Trägermaterials durchgeführt werden. Bevorzugt ist die Umsetzung ohne Zusatz von Trägermaterial.

(b) Isolierung des gebildeten Vanadium-, Phosphor-, Sauerstoff enthaltenden Katalysatorprecursors ('"VPO-Precursor"), z. B. durch Filtration oder Eindampfung.

(c) Trocknung des VPO-Precursors und bevorzugt beginnende Präformierung durch Temperung bei einer Temperatur von 250 bis 350 °C. Dem getrockneten und bevorzugt getemperten VPO-Precursor-Pulver kann nun gegebenenfalls pulverförmiges Trägermaterial und/oder ein sogenannter Porenbildner, wie beispielsweise Stearinsäure, Cellulose oder Paraffine untergemischt werden.

(d) Formgebung durch Überführung in die erfindungsgemäße Struktur. Die Formgebung erfolgt bevorzugt durch Tablettierung, vorzugsweise unter vorheriger Untermischung eines sogenannten Gleitmittels, wie etwa Graphit.

(e) Präformierung des geformten VPO-Precursors durch Erhitzen in einer Atmosphäre, welche Sauerstoff ($O_2$), Wasserstoffoxid ($H_2O$) und/oder Inertgas enthält.

Durch geeignete, an das jeweilige Katalysatorsystem angepasste Kombination von Temperaturen, Behandlungsdauern und Gasatmosphären kann die mechanische und katalytische Eigenschaft des Katalysators beeinflusst werden.

Als fünfwertige Vanadium-Verbindungen können Oxide, Säuren und anorganische und organische Salze, welche fünfwertiges Vanadium enthalten, oder deren Gemische eingesetzt werden. Bevorzugt ist der Einsatz von Vanadiumpentoxid ($V_2O_5$), Ammoniummetavanadat ($NH_4VO_3$) und Ammoniumpolyvanadat ($(NH_4)_2V_6O_{16}$), insbesondere Vanadiumpentoxid ($V_2O_5$). Die als Feststoff vorliegenden fünfwertigen Vanadium-Verbindungen werden in Form eines Pulvers, bevorzugt in einem Kornbereich von 50 bis 500 μm) eingesetzt.

Als Phosphor-Verbindungen können reduzierend wirkende Phosphor-Verbindungen, wie beispielsweise Phosphorige Säure, als auch fünfwertige Phosphor-Verbindungen, wie beispielsweise Phosphorpentoxid ($P_2O_5$), Orthophosphorsäure ($H_3PO_4$), Pyrophosphorsäure ($H_4P_2O_7$), Polyphosphorsauren der allgemeinen Formel $H_{n+2}P_nO_{3n+1}$ mit n ≥ 3 oder deren Gemische eingesetzt werden. Bevorzugt ist der Einsatz fünfwertiger Phosphor-Verbindungen. Üblicherweise gibt man den Gehalt der genannten Verbindungen und Gemische in Gew.-%, bezogen auf $H_3PO_4$ an. Bevorzugt ist der Einsatz von 80 bis 110%-iger $H_3PO_4$, besonders bevorzugt von 95 bis 110%-iger $H_3PO_4$ und ganz besonders bevorzugt von 100 bis 105%-iger $H_3PO_4$.

Als reduzierend wirkendes Lösungsmittel wird vorzugsweise ein primärer oder sekundärer, nichtcyclischer oder cyclischer, unverzweigter oder verzweigter, gesättigter Alkohol mit 3 bis 6 Kohlenstoffatomen sowie deren Gemische eingesetzt. Bevorzugt ist der Einsatz eines primären oder sekundären, unverzweigten oder verzweigten $C_3$- bis $C_6$-Alkanols oder der Einsatz von Cyclopentanol oder Cyclohexanol.

Als geeignete Alkohole seien n-Propanol (1-Propanol), Isopropanol (2-Propanol), n-Butanol (1-Butanol), sek-Butanol (2-Butanol), Isobutanol (2-Methyl-l-propanol), 1-Pentanol, 2-Pentanol, 3-Pen- tanol, 2-Methyl-l-butanol, 3-Methyl-l-butanol, 3-Methyl-2-butanol, 2, 2-Dimethyl-l-propanol, 1-Hexanol, 2-Hexanol, 3-Hexanol, 2-Methyl-1-hexanol, 3-Methyl-1-pentanol, 4-Methyl-1-pentanol, 3-Methyl-2-pentanol, 4-Methyl-2-pentanol, 2, 2-Dimethyl-1-butanol, 2, 3-Dimethyl-1-butanol, 3, 3-Dimethyl-1-butanol, 3,3-Dimethyl-2-butanol, Cyclopentanol, Cyclohexanol und deren Gemische genannt.

Ganz besonders bevorzugt sind n-Propanol (1-Propanol), n-Butanol (1-Butanol), Isobutanol (2-Methyl-1-propanol), 1-Pentanol, 2-Methyl-1-butanol, 3-Methyl-1-butanol und Cyclohexanol, insbesondere Isobutanol.

Das Zusammenfügen der Komponenten kann auf unterschiedliche Art und Weise erfolgen, beispielsweise in einem Rührkessel. Die Menge des reduzierend wirkenden Lösungsmittels sollte über der für die Reduktion des Vanadiums von der Oxidationstufe +5 auf eine Oxidationstufe im Bereich +3,5 bis +4,5 stöchiometrisch erforderlichen Menge liegen. In der Regel ist die Menge des zuzugebenden reduzierend wirkenden Lösungsmittels zumindest so bemessen, dass sie zur Aufschlämmung der fünfwertigen Vanadium-Verbindung ausreicht, welche eine intensive Vermischung mit der zuzugebenden Phosphor-Verbindung ermöglicht.

Die Aufschlämmung wird zur Umsetzung der genannten Verbindungen und Bildung des Katalysator-Precursors erhitzt. Der zu wählende Temperaturbereich ist von verschiedenen Faktoren, insbesondere der Reduktionswirkung und dem Siedepunkt der Komponenten abhängig. Im Allgemeinen stellt man eine Temperatur von 50 bis 200°C, bevorzugt von 100 bis 200°C ein. Die Umsetzung bei erhöhter Temperatur beansprucht im Allgemeinen mehrere Stunden.

Promotor-Verbindungen können zu einem beliebigen Zeitpunkt zugegeben werden. Geeignete Promotor-Verbindungen sind beispielsweise die Acetate, Acetylacetonate, Oxalate, Oxide oder Alkoxide der zuvor genannten Promotormetalle, wie etwa Cobalt acetat, Cobalt(II)-acetylacetonat, Cobalt(II)-chlorid, Molybdän(VI)-oxid, Molybdän(III)-chlorid, Eisen(III)-acetylacetonat, Eisen(III)-chlorid, Zink(II)-oxid, Zink(II)-acetylacetonat, Lithiumchlorid, Lithiumoxid, Bismut(III)-chlorid, Bismut(III)-ethylhexanoat, Nikkel(II)-ethylhexanoat, Nickel(II)-oxalat, Zirkonylchlorid, Zirkon(IV)-butoxid, Silizium(IV)-ethoxid, Niob(V)-chlorid und Niob(V)-oxid.

Nach Beendigung der vorgenannten Temperaturbehandlung wird der gebildete Katalysator-Precursor isoliert, wobei vor der Isolierung gegebenenfalls noch eine Abkühlphase sowie eine Lagerungs- oder Alterungsphase der abgekühlten Reaktionsmischung zwischengeschaltet werden können. Bei der Isolierung wird der feste Katalysator-Precursor von der flüssigen Phase abgetrennt. Geeignete Methoden sind beispielsweise Filtrieren, Dekantieren oder Zentrifugieren. Bevorzugt wird der Katalysator-Precursor durch Filtrierung isoliert.

Der isolierte Katalysator-Precursor kann ungewaschen oder gewaschen weiterverarbeitet werden. Bevorzugt wird der isolierte Katalysator-Precursor mit einem geeigneten Lösungsmittel gewaschen, um beispielsweise noch anhaftendes reduzierend wirkendes Agens (z. B. Alkohol) oder dessen Abbauprodukte zu entfernen. Geeignete Lösungsmittel sind beispielsweise Alkohole (z. B. Methanol, Ethanol, 1-Propanol, 2-Propanol), aliphatische und/oder aromatische Kohlenwasserstoffe (z. B. Pentan, Hexan, Benzine, Benzol, Toluol, Xylole), Ketone (z. B. Aceton, 2-Butanon, 3-Pentanon), Ether (z. B. 1,2-Dimethoxyethan, Tetrahydrofuran, 1,4-Dioxan) oder deren Mischungen. Wird der Katalysator-Precursor gewaschen, so werden bevorzugt 2-Propanon und/oder Methanol und besonders bevorzugt Methanol eingesetzt.

Nach der Isolierung des Katalysator-Precursors beziehungsweise nach dem Waschen wird der Feststoff im Allgemeinen getrocknet.

Die Trocknung kann unter verschiedenen Bedingungen durchgeführt werden. Im Allgemeinen führt man sie bei vermindertem Druck oder Atmosphärendruck durch. Die Trocknungstemperatur liegt in der Regel bei 30 bis 250 °C. Bevorzugt führt man die Trocknung bei einem Druck von 1 bis 30 kPa abs und einer Temperatur von 50 bis 200 °C unter sauerstoffhaltiger oder sauerstofffreier Restgasatmosphäre, wie beispielsweise Luft oder Stickstoff, durch.

In einer bevorzugten Ausführungsform zur Formgebung wird das Katalysator-Precursorpulver mit etwa 2 bis 4 Gew.-% Graphit intensiv vermischt und vorverdichtet. Die vorverdichteten Partikel werden zum Katalysatorformkörper tablettiert.

In einer weiteren Ausführungsform zur Formgebung wird das Katalysator-Precursorpulver mit etwa 2 bis 4 Gew.-% Graphit und zusätzlich mit 5 bis 40 Gew.-%, insbesondere 20 bis 25 Gew.-%, eines Porenbildners intensiv vermischt und wie oben beschrieben weiterbehandelt und geformt.

Der geformte VPO-Precursor wird durch Erhitzen in einer Atmosphäre, welche Sauerstoff ($O_2$), Wasserstoffoxid ($H_2O$) und/oder Inertgas enthält, in einem Temperaturbereich von 250 bis 600 °C präformiert (calciniert).

Als geeignete Inertgase seien beispielsweise Stickstoff, Kohlendioxid und Edelgase genannt.

Die Calcinierung kann diskontinuierlich, beispielsweise in einem Schachtofen, Hordenofen, Muffelofen oder Wärmeschrank oder kontinuierlich, beispielsweise in einem Drehrohr, Bandcalcinierofen oder Drehkugelofen durchgeführt werden. Sie kann sukzessive verschiedene Abschnitte hinsichtlich der Temperatur wie Aufheizen, Konstanthalten der Temperatur oder Abkühlen und sukzessive verschiedene Abschnitte hinsichtlich der Atmosphären wie beispielsweise sauerstoffhaltige, wasserdampfhaltige, sauerstofffreie Gasatmosphären enthalten. Geeignete Präformierungsverfahren sind beispielsweise in den Patenten US 5,137,860 und US 4,933,312 und der Offenlegungsschrift WO 95/29006 beschrieben. Besonders bevorzugt ist die kontinuierliche Calcinierung in einem Bandcalcinierofen mit mindestens zwei, beispielsweise zwei bis zehn Calcinierungszonen, welche gegebenenfalls eine unterschiedliche Gasatmosphäre und eine unterschiedliche Temperatur aufweisen. Durch geeignete, an das jeweilige Katalysatorsystem angepaßte Kombination von Temperaturen, Behandlungsdauern und Gasatmosphären kann die mechanische und katalytische Eigenschaft des Katalysators beeinflußt und somit gezielt eingestellt werden.

Bevorzugt ist eine Calcinierung, bei der man den Katalysator-Precursor

(i) in mindestens einer Calcinierungszone in einer oxidierenden Atmosphäre mit einem Sauerstoff-Gehalt von 2 bis 21 Vol.-% auf eine Temperatur von 200 bis 350 °C aufheizt und unter diesen Bedingungen bis zur Einstellung der gewünschten mittleren Oxidationsstufe des Vanadiums belässt; und

(ii) in mindestens einer weiteren Calcinierungszone in einer nicht-oxidierenden Atmosphäre mit einem Sauerstoff-Gehalt von $\leq$ 0,5 Vol.-% und einem Wasserstoffoxid-Gehalt von 20 bis 75 Vol.-% auf eine Temperatur von 300 bis 500 °C aufheizt und $\geq$ 0,5 Stunden unter diesen Bedingungen belässt.

Bei Schritt (i) wird der Katalysator-Precursor in einer oxidierend wirkenden Atmosphäre mit einem Gehalt an molekularem Sauerstoff von im Allgemeinen 2 bis 21 Vol.-% und bevorzugt von 5 bis 21 Vol.-% bei einer Temperatur von 200 bis 350 °C und bevorzugt von 250 bis 350 °C über einen Zeitraum, der wirksam ist, die gewünschte mittlere Oxidationsstufe des Vanadiums einzustellen, belassen. Im Allgemeinen setzt man bei Schritt (i) Mischungen aus Sauerstoff, Inertgasen (z.B. Stickstoff oder Argon), Wasserstoffoxid (Wasserdampf) und/oder Luft sowie Luft ein. Aus der Sicht des durch die Calcinierungszone(n) geführten Katalysator-Precursors kann die Temperatur während des Calcinierschrittes (i) konstant gehalten werden, im Mittel steigen oder fallen. Da dem Schritt (i) im Allgemeinen eine Aufheizphase vorangeschaltet ist, wird die Temperatur in der Regel zunächst ansteigen, um dann bei dem gewünschten Endwert einzupendeln. Im Allgemeinen ist daher der Calcinierungszone von Schritt (i) mindestens eine weitere Calcinierungszone zur Aufheizung des Katalysator-Precursors vorangeschaltet.

Der Zeitraum, über den die Temperung in Schritt (i) aufrecht erhalten wird, ist beim erfindungsgemäßen Verfahren bevorzugt derart zu wählen, dass sich eine mittlere Oxidationsstufe des Vanadiums auf einen Wert von +3,9 bis +4,4, bevorzugt von +4,0 bis +4,3, einstellt.

Da die Bestimmung der mittleren Oxidationsstufe des Vanadiums während der Calcinierung aus apparativen und zeitlichen Gründen nur äußerst schwierig zu bestimmen ist, ist der erforderliche Zeitraum vorteilhafterweise in Vorversuchen experimentell zu bestimmen. In der Regel dient hierzu eine Meßreihe, bei der unter definierten Bedingungen getempert wird, wobei die Proben nach unterschiedlichen Zeiten aus dem System entfernt, abgekühlt und bezüglich der mittleren Oxidationsstufe des Vanadiums analysiert werden.

Der bei Schritt (i) erforderliche Zeitraum ist im Allgemeinen abhängig von der Natur des Katalysator-Precursors, der eingestellten Temperatur und der gewählten Gasatmosphäre, insbesondere des Sauerstoff-Gehalts. Im Allgemeinen erstreckt sich der Zeitraum bei Schritt (i) auf eine Dauer von über 0,5 Stunden und bevorzugt von über 1 Stunde. Im Allgemeinen ist ein Zeitraum von bis zu 4 Stunden, bevorzugt von bis zu 2 Stunde zur Einstellung der gewünschten mittleren Oxidationsstufe ausreichend. Unter entsprechend eingestellten Bedingungen (z.B. unterer Bereich des Temperaturintervalls und/oder geringer Gehalt an molekularem Sauerstoff) kann aber auch ein Zeitraum von über 6 Stunden erforderlich sein.

Bei Schritt (ii) wird die erhaltene Katalysatorzwischenstufe in einer nicht-oxidierenden Atmosphäre mit einem Gehalt an molekularem Sauerstoff von $\leq$ 0,5 Vol.-% und an Wasserstoffoxid (Wasserdampf) von 20 bis 75 Vol.-%, bevorzugt von 30 bis 60 Vol.-% bei einer Temperatur von 300 bis 500 °C und bevorzugt von 350 bis 450 °C über einen Zeitraum von $\geq$ 0,5 Stunden, bevorzugt 2 bis 10 Stunden und besonders bevorzugt 2 bis 4 Stunden belassen. Die nicht-oxidierende Atmosphäre enthält neben dem genannten Wasserstoffoxid im Allgemeinen überwiegend Stickstoff und/oder Edelgase, wie beispielsweise Argon, wobei hierunter keine Einschränkung zu verstehen ist. Auch Gase, wie beispielsweise Kohlendioxid sind prinzipiell geeignet. Bevorzugt enthält die nicht-oxidierende Atmosphäre $\geq$ 40 Vol.-% Stickstoff. Aus der Sicht des durch die Calcinierungszone(n) geführten Katalysator-Precursors kann die Temperatur während des Calcinierschrittes (ii) konstant gehalten werden, im Mittel steigen oder fallen. Wird Schritt (ii) bei einer höheren oder tieferen Temperatur als Schritt (i) durchgeführt, so befindet sich zwischen den Schritten (i) und (ii) in der Regel eine Aufheiz- oder Abkühlphase, welche gegebenenfalls in einer weiteren Calcinierungszone implementiert ist. Um eine verbesserte Trennung zur sauerstoffhaltigen Atmosphäre des Schrittes (i) zu ermöglichen, kann diese weitere Calcinierungszone zwischen (i) und (ii) beispielsweise zur Spülung mit Inertgas, wie beispielsweise Stickstoff, gespült werden. Bevorzugt wird Schritt (ii) bei einer um 50 bis 150 °C höheren Temperatur als Schritt (i) durchgeführt.

Im Allgemeinen umfasst die Calcinierung einen weiteren, zeitlich nach Schritt (ii) durchzuführenden Schritt (iii), bei dem man den calcinierten Katalysator-Precursor in einer Inertgas-Atmosphäre auf eine Temperatur von $\leq$ 300 °C, bevorzugt von $\leq$ 200 °C und besonders bevorzugt von $\leq$ 150 °C abkühlt.

Vor, zwischen und/oder nach den Schritten (i) und (ii), beziehungsweise (i), (ii) und (iii) sind bei der Calcinierung nach dem erfindungsgemäßen Verfahren weitere Schritte möglich. Ohne limitierend zu wirken seien als weitere Schritte beispielsweise Änderungen in der Temperatur (Aufheizen, Abkühlen), Änderungen in der Gasatmosphäre (Umstellung der Gasatmosphäre), weitere Haltezeiten, Überführungen der Katalysatorzwischenstufe in andere Apparate oder Unterbrechungen des gesamten Calciniervorgangs genannt.

Da der Katalysator-Precursor in der Regel vor Beginn der Calcinierung eine Temperatur von < 100 °C besitzt, ist dieser vor Schritt (i) üblicherweise aufzuheizen. Das Aufheizen kann unter Anwendung verschiedener Gasatmosphären durchgeführt werden. Vorzugsweise wird das Aufheizen in einer oxidierend wirkenden Atmosphäre, wie unter Schritt (i) definiert, oder einer Inertgas-Atmosphäre, wie unter Schritt (iii) definiert, durchgeführt. Auch ein Wechsel der Gasatmosphäre während der Aufheizphase ist möglich. Besonders bevorzugt ist das Aufheizen in der oxidierend wirkenden Atmosphäre, welche auch in Schritt (i) angewendet wird.

Weiterhin ist Gegenstand der Erfindung ein Verfahren zur Herstellung von Maleinsäureanhydrid durch heterogenkatalytische Gasphasenoxidation eines Kohlenwasserstoffs mit mindestens vier Kohlenstoffatomen mit Sauerstoff enthaltenden Gasen unter Einsatz des erfindungsgemäßen Katalysators. Als Reaktoren werden im Allgemeinen Rohrbündelreaktoren eingesetzt. Geeignete Rohrbündelreaktoren sind beispielsweise in der EP-B 1 261 424 beschrieben.

Als Kohlenwasserstoffe sind im erfindungsgemäßen Verfahren aliphatische und aromatische, gesättigte und ungesättigte Kohlenwasserstoffe mit mindestens vier Kohlenstoffatomen geeignet, beispielsweise 1,3-Butadien, 1-Buten, 2-cis-Buten, 2-trans-Buten, n-Butan, $C_4$-Gemisch, 1,3-Pentadien, 1,4-Pentadien, 1-Penten, 2-cis-Penten, 2-trans-Penten, n-Pentan, Cyclopentadien, Dicyclopentadien, Cyclopenten, Cyclopentan, $C_5$-Gemisch, Hexene, Hexane, Cyclohexan und Benzol. Bevorzugt eingesetzt werden Propan, 1-Buten, 2-cis-Buten, 2-trans-Buten, n-Butan, Benzol oder deren Mischungen, insbesondere Propan, n-Butan oder Benzol. Besonders bevorzugt ist der Einsatz von n-Butan, beispielsweise als reines n-Butan oder als Komponente in n-Butan-haltigen Gasen und Flüssigkeiten. Das verwendete n-Butan kann beispielsweise aus dem Erdgas, aus Steamcrackern oder FCC-Crackern stammen.

Die Zugabe des Kohlenwasserstoffs erfolgt im Allgemeinen mengengeregelt, d. h. unter stetiger Vorgabe einer definierten Menge pro Zeiteinheit. Der Kohlenwasserstoff kann in flüssiger oder gasförmiger Form dosiert werden. Bevorzugt ist die Dosierung in flüssiger Form mit anschließender Verdampfung vor Eintritt in die Rohrbündelreaktor-Einheit.

Als Oxidationsmittel werden Sauerstoff enthaltende Gase, wie beispielsweise Luft, synthetische Luft, ein mit Sauerstoff angereichertes Gas oder auch sogenannter "reiner", z. B. aus der Luftzerlegung stammender Sauerstoff eingesetzt. Auch das Sauerstoffenthaltende Gas wird mengengeregelt zugegeben.

Das erfindungsgemäße Verfahren wird bei einer Temperatur von 250 bis 500 °C durchgeführt. Unter der genannten Temperatur ist, unabhängig von der Art des Reaktors, jeweils die mittlere Temperatur des Wärmeträgermediums zu verstehen. Im Falle der Verwendung von n-Butan als Kohlenwasserstoff-Edukt wird das erfindungsgemäße Verfahren bevorzugt bei einer Temperatur von 380 bis 460 °C und besonders bevorzugt 380 bis 440 °C durchgeführt. Bei der Verwendung von Propan wird das erfindungsgemäße Verfahren bevorzugt zwischen 250 und 350 °C ausgeführt. Bei der Verwendung von Benzol wird das erfindungsgemäße Verfahren bevorzugt zwischen 330 und 450 °C ausgeführt.

Das erfindungsgemäße Verfahren wird vorteilhaft isotherm, mit einer über die Reaktorlänge ansteigender Temperaturführung oder mit einer Kombination aus über die Reaktorlänge ansteigender Temperaturführung und isothermer Fahrweise durchgeführt.

Das erfindungsgemäße Verfahren wird vorteilhaft bei einem Sauerstoffpartialdruck von 0,6 bar bis 50 bar, bevorzugt 2 bar bis 50 bar, besonders bevorzugt 3 bar bis 50 bar, insbesondere 4 bar bis 50 bar durchgeführt.

Die Kohlenwasserstoff-Konzentration des der Reaktor-Einheit zugeführten Eingangsstroms beträgt 0,5 bis 10 Vol.-%, bevorzugt 0,8 bis 10 Vol.-%, besonders bevorzugt 1 bis 10 Vol.-% und ganz besonders bevorzugt 2 bis 10 Vol.-%.

Der Kohlenwasserstoff-Umsatz pro Reaktordurchgang beträgt 40 bis 100 %, bevorzugt 50 bis 95 %, besonders bevorzugt 70 bis 95 % und insbesondere 85 bis 95 % des Kohlenwasserstoffs aus dem Eingangsstrom.

Beim erfindungsgemäßen Verfahren stellt man über die Menge des Eingangsstroms in der Reaktor-Einheit eine GHSV (gas hourly space velocity) von bevorzugt 2000 bis 10000 $h^{-1}$ und besonders bevorzugt 3000 bis 8000 $h^{-1}$, bezogen auf das auf 0 °C und 0,1013 MPa abs normierte Volumen des zugeführten Eingangsstroms und bezogen auf das Reaktionsvolumen, das mit Katalysator befüllt ist bzw. dessen geometrischen Oberfläche beschichtet ist.

Das erfindungsgemäße Verfahren kann in zwei bevorzugten Verfahrensvarianten, der Variante mit "geradem Durchgang" und der Variante mit "Rückführung" durchgeführt werden. Beim "geraden Durchgang" wird aus dem Reaktoraustrag Maleinsäureanhydrid und gegebenenfalls oxygenierte Kohlenwasserstoff-Nebenprodukte entfernt und das verbleibende Gasgemisch ausgeschleust und gegebenenfalls thermisch verwertet. Bei der "Rückführung" wird aus dem Reaktoraustrag ebenfalls Maleinsäureanhydrid und gegebenenfalls oxygenierte Kohlenwasserstoff-Nebenprodukte entfernt, das verbleibende Gasgemisch, welches nicht-umgesetzten Kohlenwasserstoff enthält, ganz oder teilweise zum Reaktor rückgeführt. Eine weitere Variante der "Rückführung" ist die Entfernung des nicht-umgesetzten Kohlenwasserstoffs und dessen Rückführung zum Reaktor.

Die Umsetzungsprodukte bzw. der Produktstrom können gegebenenfalls durch Zugabe von unter Reaktionsbedingungen inerten Stoffe wie beispielsweise Wasser oder Stickstoff am Ende des Reaktors oder am Reaktorausgang verdünnt werden, so dass ein nicht-explosiver Produktstrom erhalten wird. Ferner kann vorteilhaft ein nicht-explosiver Produktstrom durch eine Druckstufe erreicht werden. Dieser Produktstrom lässt sich dann mit den konventionellen Aufarbeitungseinheiten aufbereiten.

Unter Verwendung von n-Butan wird zur Gewährung einer langen Katalysatorstandzeit und weiteren Erhöhung von Umsatz, Selektivität, Ausbeute, Katalysator-Belastung und Raum/Zeit-Ausbeute dem Gas beim erfindungsgemäßen Verfahren vorteilhaft eine flüchtige Phosphorverbindung zugeführt. Ihre Konzentration beträgt zu Beginn, d.h. am Reaktoreingang 0,2 bis 20 Volumen-ppm der flüchtigen Phosphorverbindungen bezogen auf das Gesamtvolumen des Gases am Reaktoreingang. Bevorzugt ist ein Gehalt 0,5 bis 5 Volumen-ppm. Als flüchtige Phosphorverbindungen sind all jene Phosphorenthaltende Verbindungen zu verstehen, welche in der gewünschten Konzentration unter den Einsatzbedingungen gasförmig vorliegen. Bevorzugt werden als flüchtige Phosphorverbindung Triethylphosphat oder Trimethylphosphat eingesetzt.

Die Erfindung wird durch die beigefügten Zeichnungen und die folgenden Beispiele näher veranschaulicht.

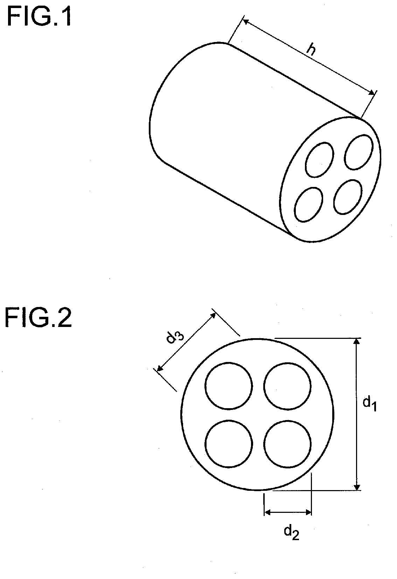

Fig. 1 zeigt die Ansicht eines erfindungsgemäßen Katalysatorformkörpers mit vier Innenbohrungen.

Fig. 2 zeigt eine Draufsicht auf den in Fig. 1 dargestellten Katalysatorformkörper.

Definitionen

Die in dieser Schrift verwendeten Größen sind, falls nicht anders erwähnt, wie folgt definiert:

geometrische Oberfläche $A_{geo}$ = geometrische Oberfläche der Formkörper unter Zugrundelegung der geometrischen Grössen $d_1$, h und $d_2$ [$mm^2$]

geometrisches Volumen $V_{geo}$ = geometrisches Volumen der Formkörper unter Zugrundelegung der geometrischen Grössen $d_1$, h und $d_2$ [$mm^3$]

theoretisches Volumen Vollzylinder $V_{overall}$ = theoretisches Volumen eines entsprechenden Vollzylinders mit Höhe h und äußerem Durchmesser $d_1$ [$mm^3$]

$X_{n\text{-Butan}}$ = Butan-Konzentration des Eingangsstroms

$X_{n\text{-Butan}}$ = n-Butan-Umsatz

$X_{TEP}$ = Triethylphosphat-Konzentration des Eingangsstroms

$X_{H2O}$ = Wasserdampf-Konzentration des Eingangsstroms

GHSV = Menge des Eingangsstroms, bezogen auf das auf 0 °C und 0,1013 MPa abs normierte Volumen des zugeführten Eingangsstroms und bezogen auf das Reaktionsvolumen, das mit Katalysator befüllt ist

Rohrfüllgewicht = Schüttdichte des Katalysators im Reaktionsrohr

$$\text{Selektivität S (Acrylsäure)} = n_{Acrylsäure,Reaktor,aus}/(n_{Butan,Reaktor,ein} - n_{Butan,Reaktor,aus})$$

$$\text{Selektivität S (Essigsäure)} = n_{Essigsäure,Reaktor,aus}/(n_{Butan,Reaktor,ein} - n_{Butan,Reaktor,aus})$$

Bestimmung des Rest-Isobutanolgehalts im getrockneten Katalysator-Precursor

Zur Bestimmung des Rest-Isobutanolgehalts wurden etwa 4 g des getrockneten pulverförmigen Katalysator-Precursors und etwa 10 g N,N-Dimethylformamid in eine beheizbare Rührapparatur mit Rückflusskühler genau eingewogen. Anschließend wurde unter Rühren auf Siedetemperatur aufgeheizt und 30 Minuten unter diesen Bedingungen belassen. Nach der Abkühlung wurde die Suspension filtriert und im Filtrat der Gehalt an Isobutanol durch Gaschromatographie quantitativ erfasst. Der Rest-Isobutanolgehalt wurde dann aus der ermittelten Konzentration an Isobutanol im N,N-Dimethylformamid und der eingewogenen Mengen an N,N-Dimethylformamid und Katalysator-Precursor berechnet.

Bestimmung der Seitendruckfestigkeit der Hohlzylinder

Zur Bestimmung der Seitendruckfestigkeit wurden in nacheinander folgenden Messungen die Katalysatorformkörper mit der gerundeten Seitenfläche jeweils auf die plane Metall-Auflageplatte einer entsprechenden Messeinrichtung gestellt. Die beiden planparallelen Deckelflächen befanden sich somit in vertikaler Richtung. Nun wurde ein planer Metall-Stempel von oben mit einer Vorschubgeschwindigkeit von 1,6 mm/min auf den Katalysatorformkörper zugefahren und der zeitliche Verlauf der Krafteinwirkung auf den Katalysatorformkörper bis zu dessen Bruch aufgezeichnet. Die Seitendruckfestigkeit des einzelnen Katalysatorformkörper entspricht der maximal eingewirkten Kraft.

Bestimmung des spezifischen Porenvolumens

Das spezifische Porenvolumen wurde durch Quecksilber-Porosimetrie gemäß der DIN 66133 bestimmt.

Herstellung des Katalysator-Precursors

In einem mit Stickstoff inertisierten, über Druckwasser außenbeheizbaren 8 m³-Stahl/Email-Rührkessel mit Strombrechern wurden 6,1 m³ Isobutanol vorgelegt. Nach Inbetriebnahme des dreistufigen Impellerrührers wurde das Isobutanol unter Rückfluss auf 90 °C aufgeheizt. Bei dieser Temperatur wurde nun über die Förderschnecke mit der Zugabe von 736 kg Vanadiumpentoxid begonnen. Nachdem nach etwa 20 Minuten etwa 2/3 der gewünschten Menge an Vanadiumpentoxid zugegeben wurden, wurde bei weiterer Zugabe an Vanadiumpentoxid mit der Einpumpung von 900 kg 105%-iger Phosphorsäure begonnen. Zur Reinigung der Pumpe wurden weitere 0,2 m³ Isobutanol nachge-pumpt. Anschliessend wurde das Reaktionsgemisch unter Rückfluss auf etwa 100 bis 108 °C erhitzt und unter diesen Bedingungen 14 Stunden belassen. Im Anschluss daran wurde die heiße Suspension in eine zuvor mit Stickstoff inertisierte und beheizte Druckfilternutsche abgelassen und bei einer Temperatur von etwa 100 °C bei einem Druck oberhalb der Filternutsche von bis zu 0,35 MPa abs abfiltriert. Der Nutschkuchen wurde durch stetiges Einleiten von Stickstoff bei 100 °C und unter Rühren mit einem mittig angeordneten, in der Höhe verstellbaren Rührer innerhalb von etwa einer Stunde trockengeblasen. Nach dem Trockenblasen wurde auf etwa 155 °C aufgeheizt und auf einen Druck von 15 kPa abs (150 mbar abs) evakuiert. Die Trocknung wurde bis zu einem Rest-Isobutanolgehalt von < 2 Gew.-% im getrockneten Katalysator-Precursor durchgeführt.

Das erhaltene, getrocknete Pulver wurde nun 2 Stunden unter Luft in einem Drehrohr mit einer Länge von 6,5 m, einem Innendurchmesser von 0,9 m und innenliegenden spiralförmigen Wendeln getempert. Die Drehzahl des Drehrohres betrug 0,4 U/min. Das Pulver wurde in einer Menge von 60 kg/h in das Drehrohr gefördert. Die Luftzufuhr betrug 100 m³/h. Die direkt an der Außenseite des Drehrohrs gemessenen Temperaturen der fünf gleichlangen Heizzonen betrugen 250 °C, 300 °C, 340 °C, 340 °C und 340 °C. Nach dem Abkühlen auf Raumtemperatur wurde der Katalysator-Precursor mit 1 Gew.-% Graphit innig vermischt und in einem Walzen-Kompaktor kompaktiert. Das Feingut im Kompaktat mit einer Partikelgrösse < 400 μm wurde abgesiebt und erneut der Kompaktierung zugeführt. Das Grobgut mit einer Partikelgrösse 400 μm wurde mit weiteren 2 Gew.-% Graphit innig vermischt. Im Folgenden wird dieses als "Katalysatorprecursor-Pulver" bezeichnet.

Herstellung der Katalysatoren A, B und C

Zur Herstellung der Katalysatoren A und C wurde das Katalysatorprecursor-Pulver mit 20 Gew.-% Malonsäure gemischt. Das Katalysatorprecursor-Pulver bzw. das Gemisch mit Malonsäure wurde in einer Tablettiermaschine zu 6,5x4,2x3,7 mm Hohlzylindern (äußerer Durchmesser x Höhe x Durchmesser des inneren Lochs) (Katalysator A, nicht erfindungsgemäß) bzw.

Zylindern 6,5x4,2 (äußerer Durchmesser x Höhe) mit vier durchgehenden Löchern mit einem Durchmesser von 1,85 (Katalysatoren B und C) tablettiert. Während beim Hohlzylinder Presskräfte von etwa 10 kN bei der Tablettierung eingestellt werden, reichen beim Formkörper mit den vier Innenbohrungen etwa 8 kN aus.

Die geometrischen Eigenschaften der Formkörper sind wie folgt:

| Katalysatoren | | A | B,C |
|---|---|---|---|
| $d_1$ | [mm] | 6,5 | 6,5 |
| h | [mm] | 4,2 | 4,2 |
| $d_2$ | [mm] | 3,7 | 1,85 |
| Anzahl Bohrungen | | 1 | 4 |
| | | | |
| Äußere Mantelfläche | [mm²] | 86 | 86 |
| Stirnflächen | [mm²] | 22 | 22 |
| Innere Mantelfläche(n) | [mm²] | 49 | 98 |
| $A_{geo}$ | [mm²] | 157 | 206 |
| $V_{geo}$ | [mm³] | 94 | 94 |
| $A_{geo}/V_{geo}$ | [mm⁻¹] | 1,67 | 2,18 |
| $V_{overall}$ | [mm³] | 139 | 139 |
| $V_{geo}/V_{overall}$ | % | 68 | 68 |

Die tablettierten Katalysatorprecursor-Proben (Grünlinge) wurden nacheinander in einen Bandcalcinierer gegeben und wie folgt calciniert, wobei die Verweilzeit in jeder Calcinierzone jeweils etwa 1,78 h betrug:

Präfomierarameter Katalysatoren A und C (mit Malonsäure)

| Zone | Temperatur | zugeführtes Frischgas |
|---|---|---|
| Calcinierzone 1 | 150 °C | Luft |
| Calcinierzone 2 | 180 °C | Luft |
| Calcinierzone 3 | 280 °C | Luft, $N_2/H_2O$-Dampf (5 Vol% $O_2$) |
| Calcinierzone 4 | 325 °C | Luft, $N_2/H_2O$-Dampf (5 Vol% $O_2$) |
| Übergangszone | Abkühlen auf 200 °C | |
| Calcinierzone 5 | 335 °C | $N_2$ |
| Calcinierzone 6 | 400 °C | $N_2/H_2O$-Dampf (1:1) |
| Calcinierzone 7 | 425 °C | $N_2/H_2O$-Dampf (1:1) |
| Calcinierzone 8 | 355 °C | $N_2$ |

Präfomierparameter Katalysator B (ohne Porenbildner)

| Zone | Temperatur | zugeführtes Frischgas |
|---|---|---|
| Calcinierzone 1 | 140 °C | Luft |
| Calcinierzone 2 | 140 °C | Luft |
| Calcinierzone 3 | 260 °C | Luft |
| Calcinierzone 4 | 300 °C | Luft |
| Übergangszone | Abkühlen auf 200 °C | Luft |
| Calcinierzone 5 | 335 °C | $N_2$ |
| Calcinierzone 6 | 400 °C | $N_2/H_2O$-Dampf (1:1) |
| Calcinierzone 7 | 425 °C | $N_2/H_2O$-Dampf (1:1) |
| Calcinierzone 8 | 355 °C | $N_2$ |

Katalytische Tests

Die Versuchsanlage war ausgestattet mit einer Zufuhr-Einheit und einem Reaktorrohr. Der Ersatz eines Rohrbündelreaktors durch ein Reaktorrohr ist im Labor- oder Technikumsmaßstab sehr gut möglich, sofern die Abmessungen des Reaktorrohres im Bereich eines technischen Reaktorrohres liegen. Die Anlage wurde im "geraden Durchgang" betrieben.

Der Kohlenwasserstoff wurde mengengeregelt in flüssiger Form über eine Pumpe zugegeben. Als sauerstoffhaltiges Gas wurde Luft mengengeregelt zugegeben. Triethylphosphat (TEP) wurde ebenfalls mengengeregelt, gelöst in Wasser, in flüssiger Form zugegeben.

Die Rohrbündelreaktor-Einheit bestand aus einem Rohrbündelreaktor mit einem Reaktorrohr. Die Länge des Reaktorrohrs betrug 6,5 m, der Innendurchmesser 22,3 mm. Innerhalb des Reaktorrohres befand sich in einem Schutzrohr mit 6 mm Außendurchmesser ein Multi-Thermoelement mit 20 Temperaturmessstellen. Die Temperierung des Reaktors erfolgte durch einen Wärmeträger-Kreislauf mit einer Länge von 6,5 m. Als Wärmeträgermedium wurde eine Salzschmelze eingesetzt. Das Reaktorrohr wurde von dem Reaktionsgasgemisch von oben nach unten durchströmt. Die oberen 0,2 m des 6,5 m langen Reaktorrohres blieben ungefüllt. Als Nächstes folgte eine 0,3 m lange Vorheizzone, welche mit Steatit-Formkörpern als Inertmaterial gefüllt war. Im Anschluss an die Vorheizzone folgte die Katalysatorschüttung, welche insgesamt 2173 mL Katalysator enthielt.

Direkt nach der Rohrbündelreaktor-Einheit wurde gasförmiges Produkt entnommen und der gaschromatographischen on-line Analytik zugeführt. Der Hauptstrom des gasförmigen Reaktoraustrags wurde aus der Anlage ausgeschleust.

Die Reaktionsbedingungen für die katalytische Testung waren wie folgt: $x_{n\text{-Butan}}$ = 2 Vol.-%, GHSV = 2000 $h^{-1}$, $P_{ein}$ = 2,3 barg, $X_{n\text{-Butan}}$ = 85 %, $x_{TEP}$ = 2,25 - 2,5 Vol-ppm, $x_{H2O}$ = 3 Vol-%.

Die Messungen erfolgten nach einer Mindestlaufzeit des Katalysators von 370 h.

Tabelle: Charakterisierungsergebnisse der drei Katalysatoren.

| Katalysator | | A | B | C |
|---|---|---|---|---|
| Grünling | | | | |
| Seitendruckfestigkeit Engstelle* | [N] | 14,1 | 5,6 | 11,8 |
| Seitendruckfestigkeit Steg** | [N] | | 59 | 61 |
| Dichte | [g/ml] | 1,69 | 1,55 | 1,57 |
| Präformierung | | | | |
| $T_{max}$ | [°C] | 390 | 380 | 390 |
| $t_{Tmax}$ | [min] | 1 | 0 | 1 |
| Katalysator | | | | |
| Seitendruckfestigkeit Engstelle* | [N] | 11,4 | 8 | 8 |

(fortgesetzt)

| Katalysator | | A | B | C |
|---|---|---|---|---|
| Seitend ruckfestigkeit Steg** | [N] | | 35 | 29 |
| Dichte | [g/ml] | 1,35 | 1,44 | 1,29 |
| $V_{Ox}$ | | 4,18 | 4,18 | 4,16 |
| Porenvolumen (PV) | [ml/g] | 0,368 | 0,336 | 0,408 |
| Katalytische Testung | | | | |
| Rohrfüllgewicht | [g/l] | 442 | 458 | 417 |
| MSA-Ausbeute | [mol%] | 54,5 | 57,9 | 58,9 |
| MSA-Ausbeute | [m/m %] | 95,5 | 97,9 | 99,0 |
| Selektivität Acrylsäure | [mol%] | 1,02 | 1,16 | 1,12 |
| Selektivität Essigsäure | [mol%] | 1,22 | 1,21 | 1,34 |
| Salzbadtemperatur | [°C] | 406 | 410 | 405 |

\* Seitendruckfestigkeit Engstelle: Der Formkörper wurde so in die Apparatur zur Bestimmung der Seitendruckfestigkeit eingelegt, dass der in Richtung der einwirkenden Kraft liegende Formkörperdurchmesser zwei volle Innenbohrungen umfasste.

\*\* Seitendruckfestigkeit Steg: Der Formkörper wurde so in die Apparatur zur Bestimmung der Seitendruckfestigkeit eingelegt, dass der in Richtung der einwirkenden Kraft liegende Formkörperdurchmesser keine Innenbohrungen umfasste.

Die Ergebnisse zeigen, dass die Katalysatoren B und C mit vier Innenbohrungen zu einer deutlichen Steigerung der MSA-Ausbeute führten, wobei der Einsatz eines Porenbildners (Katalysator C) zu einer weiteren Ausbeutesteigerung führte. Überraschend ist, dass der Katalysator C das geringste Rohrfüllgewicht zeigte, d. h. eine höhere Ausbeute mit einer geringeren Katalysatormenge erreicht wurde. Die erreichten Seitendruckfestigkeiten der Katalysatoren B und C sind für die praktischen Erfordernisse ausreichend.

**Patentansprüche**

1. Katalysatorformkörper zur Herstellung von Maleinsäureanhydrid durch Gasphasenoxidation eines Kohlenwasserstoffs mit mindestens vier Kohlenstoffatomen, umfassend eine katalytisch aktive Masse, die Vanadium, Phosphor und Sauerstoff enthält, wobei der Katalysatorformkörper einen im wesentlichen zylindrischen Körper mit einer Längsachse aufweist,
**dadurch gekennzeichnet,**
**dass** der zylindrische Körper vier zur Zylinderachse des Körpers im wesentlichen parallele, durchgehende Innenbohrungen aufweist, die Summe der Mantelflächen der Innenbohrungen gleich oder größer als die äußere Mantelfläche des zylindrischen Körpers ist und sowohl der kleinste Abstand der Innenbohrungen untereinander als auch der kleinste Abstand der Innenbohrungen zur äußeren Mantelfläche des Körpers jeweils wenigstens 6% des Durchmessers des zylindrischen Körpers beträgt.

2. Katalysatorformkörper gemäß Anspruch 1, **dadurch gekennzeichnet, dass** die Zentralachsen der Innenbohrungen äquidistant auf einem Zylindermantel liegen, der konzentrisch zum Mantel des zylindrischen Körpers ist.

3. Katalysatorformkörper gemäß einem der vorhergehenden Ansprüche, **dadurch gekennzeichnet, dass** das Verhältnis der geometrischen Oberfläche $A_{geo}$ zum geometrischen Volumen $V_{geo}$ mindestens 2,0 mm$^{-1}$ beträgt.

4. Katalysator gemäß einem der Ansprüche 1 bis 3, **dadurch gekennzeichnet, dass** das Verhältnis des geometrischen Volumens $V_{geo}$ des zylindrischen Körpers zum theoretischen Volumen $V_{overall}$ eines entsprechenden Vollzylinders mit gleicher Höhe h und gleichem äußeren Durchmesser $d_1$ höchstens 0,85 beträgt.

5. Katalysatorformkörper gemäß einem der vorhergehenden Ansprüche, **dadurch gekennzeichnet, dass** der äußere

Durchmesser $d_1$ des zylindrischen Körpers 3 bis 10 mm, die Höhe h des zylindrischen Körpers 1 bis 10 mm und der Durchmesser jeder Innenbohrung $d_2$ 0,5 bis 4 mm beträgt.

6. Katalysatorformkörper gemäß einem der vorhergehenden Ansprüche, **dadurch gekennzeichnet, dass** der Formkörper porös ist.

7. Katalysatorformkörper gemäß Anspruch 6, **dadurch gekennzeichnet, dass** der Formkörper ein spezifisches Porenvolumen, bestimmt durch Quecksilberporosimetrie gemäß DIN 66133, von mindestens 0,30 ml/g aufweist.

8. Verfahren zur Herstellung von Maleinsäureanhydrid, **dadurch gekennzeichnet, dass** man einen Kohlenwasserstoff mit mindestens vier Kohlenstoffatomen in Gegenwart eines Sauerstoff enthaltenden Gases mit einer Schüttung von Katalysatorformkörpern gemäß einem der vorhergehenden Ansprüche in Kontakt bringt.


**Claims**

1. A shaped catalyst body for preparing maleic anhydride by gas-phase oxidation of a hydrocarbon having at least four carbon atoms using a catalytically active composition comprising vanadium, phosphorus and oxygen, where the shaped catalyst body has an essentially cylindrical body having a longitudinal axis, wherein the cylindrical body has four parallel internal holes which are essentially parallel to the cylinder axis of the body and go right through the body, the sum of the surfaces of the internal holes is equal to or greater than the external surface of the cylindrical body and both the smallest distance between the internal holes and the smallest distance between the internal holes and the outer cylindrical surface of the body is in each case at least 6% of the diameter of the cylindrical body.

2. The shaped catalyst body according to claim 1, wherein the central axes of the internal holes are located equidistantly on a cylindrical surface which is concentric with the surface of the cylindrical body.

3. The shaped catalyst body according to any of the preceding claims, wherein the ratio of the geometric surface $A_{geo}$ to the geometric volume $V_{geo}$ is at least 2.0 mm$^{-1}$.

4. The catalyst according to any of claims 1 to 3, wherein the ratio of the geometric volume $V_{geo}$ of the cylindrical body to the theoretical volume $V_{overall}$ of a corresponding solid cylinder having the same height h and the same external diameter $d_1$ is not more than 0.85.

5. The shaped catalyst body according to any of the preceding claims, wherein the external diameter $d_1$ of the cylindrical body is from 3 to 10 mm, the height h of the cylindrical body is from 1 to 10 mm and the diameter of each internal hole $d_2$ is from 0.5 to 4 mm.

6. The shaped catalyst body according to any of the preceding claims, wherein the shaped body is porous.

7. The shaped catalyst body according to claim 6, wherein the shaped body has a specific pore volume, determined by mercury porosimetry in accordance with DIN 66133, of at least 0.30 ml/g.

8. A process for preparing maleic anhydride, wherein a hydrocarbon having at least four carbon atoms is brought into contact with a bed of shaped catalyst bodies according to any of the preceding claims in the presence of an oxygen-comprising gas.


**Revendications**

1. Corps moulé catalytique pour la fabrication d'anhydride de l'acide maléique par oxydation en phase gazeuse d'un hydrocarbure contenant au moins quatre atomes de carbone, comprenant une masse catalytiquement active, qui contient du vanadium, du phosphore et de l'oxygène, le corps moulé catalytique comprenant un corps essentiellement cylindrique ayant un axe longitudinal, **caractérisé en ce que**

le corps cylindrique comprend quatre alésages intérieurs continus essentiellement parallèles à l'axe du cylindre du corps, la somme des surfaces d'enveloppe des alésages intérieurs étant supérieure ou égale à la surface d'enveloppe extérieure du corps cylindrique, et aussi bien le plus petit écart entre les alésages intérieurs que le plus petit écart entre les alésages intérieurs et la surface d'enveloppe extérieure du corps étant à chaque fois d'au moins 6 % du diamètre du corps cylindrique.

**2.** Corps moulé catalytique selon la revendication 1, **caractérisé en ce que** les axes centraux des alésages intérieurs sont équidistants d'une enveloppe cylindrique qui est concentrique à l'enveloppe du corps cylindrique.

**3.** Corps moulé catalytique selon l'une quelconque des revendications précédentes, **caractérisé en ce que** le rapport entre la surface géométrique $A_{géo}$ et le volume géométrique $V_{géo}$ est d'au moins 2,0 mm$^{-1}$.

**4.** Catalyseur selon l'une quelconque des revendications 1 à 3, **caractérisé en ce que** le rapport entre le volume géométrique $V_{géo}$ du corps cylindrique et le volume théorique $V_{total}$ d'un cylindre plein correspondant ayant la même hauteur h et le même diamètre extérieur $d_1$ est d'au plus 0,85.

**5.** Corps moulé catalytique selon l'une quelconque des revendications précédentes, **caractérisé en ce que** le diamètre extérieur $d_1$ du corps cylindrique est de 3 à 10 mm, la hauteur h du corps cylindrique est de 1 à 10 mm et le diamètre de chaque alésage intérieur $d_2$ est de 0,5 à 4 mm.

**6.** Corps moulé catalytique selon l'une quelconque des revendications précédentes, **caractérisé en ce que** le corps moulé est poreux.

**7.** Corps moulé catalytique selon la revendication 6, **caractérisé en ce que** le corps moulé présente un volume poreux spécifique, déterminé par porosimétrie au mercure selon DIN 66133, d'au moins 0,30 ml/g.

**8.** Procédé de fabrication d'anhydride de l'acide maléique, **caractérisé en ce qu'**un hydrocarbure contenant au moins quatre atomes de carbone est mis en contact en présence d'un gaz contenant de l'oxygène avec un garnissage de corps moulés catalytiques selon l'une quelconque des revendications précédentes.

FIG.1

FIG.2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4283307 A
- US 5168090 A
- WO 0168245 A
- WO 03078057 A
- WO 2007051602 A
- EP 1120390 A
- JP 6170239 A
- WO 2008087116 A
- US 5275996 A
- US 5641722 A

- US 5137860 A
- US 5095125 A
- US 4933312 A
- WO 9712674 A
- WO 9526817 A
- US 5296436 A
- US 5158923 A
- US 4795818 A
- WO 9529006 A
- EP 1261424 B